Europäisches Patentamt

(19) European Patent Office

Office européen des brevets

(11) Publication number: **0 109 197**
A2

# (12) EUROPEAN PATENT APPLICATION

(21) Application number: **83306273.0**

(51) Int. Cl.³: **A 61 L 15/04,** A 61 L 15/03

(22) Date of filing: **17.10.83**

(30) Priority: 18.10.82 US 435148
16.06.83 US 505008

(43) Date of publication of application: **23.05.84**
Bulletin 84/21

(84) Designated Contracting States: **AT BE CH DE FR GB IT
LI NL SE**

(71) Applicant: **Johnson & Johnson Products Inc., 501 George
Street, New Brunswick New Jersey 08903 (US)**

(72) Inventor: **Pines, Eli, 1316 Johnston Drive, Watchung New
Jersey 07060 (US)**
Inventor: **Cunningham, Timothy Joseph, 76 Park Avenue,
Flemington New Jersey 08822 (US)**

(74) Representative: **Jones, Alan John et al, CARPMAELS &
RANSFORD 43 Bloomsbury Square, London, WC1A 2RA
(GB)**

(54) Synthetic absorbable hemostat.

(57) Absorbable hemostatic fabric materials are constructed of filaments comprising homopolymers or copolymers of lactide and glycolide. The fabrics are exposed to from about 5 to 50 Mrads radiation as required to reduce the in vivo absorption time of the material to about 14 to 28 days. Thrombin or other coagulating agents may be incorporated in the fabrics to improve hemostatic performance.

EP 0 109 197 A2

# SYNTHETIC ABSORBABLE HEMOSTAT

## FIELD OF THE INVENTION

The present invention relates to surgical materials for control of bleeding, and more particularly, to fabric hemostatic materials comprised of synthetic absorbable polymers.

## BACKGROUND OF THE INVENTION

The control of bleeding is a serious problem in certain surgical procedures and in various types of emergency wounds. Bleeding from the kidney, brain, or liver or the persistent oozing from severed capillaries and veins, for example, is particularly difficult to control by conventional means such as suturing or ligature and in many cases is serious enough to endanger life. Surgical hemostats consisting of conventional gauze pads or similar articles impregnated with a hemostatic material such as ferric chloride, thrombin or the like, have been used for many years to arrest bleeding. Hemostats of this type cannot be left in situ in a closed wound, however, since foreign body tissue reaction would result. This is a serious disadvantage inasmuch as removal of the hemostat from the bleeding site frequently disrupts any blood clot which has formed and causes renewed bleeding.

The need for biologically compatible absorbable hemostatic materials which could be left in place in a closed wound led to the development of the oxidized cellulose absorbable hemostat as described in U.S. Patent No. 3,364,200, and the finely divided fibrous collagen hemostat as described in U.S. Patent No. 3,742,955. An absorbable hemostatic sponge prepared from a skin gelatin is also described in U.S. Patent No. 2,465,357.

iJ 1128

The prior art absorbable hemostats are derived from animal or plant materials and are accordingly subject to certain variabilities in composition and properties inherent with such materials. Additionally, the gelatin sponge is ineffective against profusely bleeding wounds such as a splenic incision, and the collagen fluff becomes sticky and difficult to handle.

Collagen sponge hemostatic material such as that described in U.S. Patent No. 4,320,201 is an effective hemostat against profusely bleeding wounds and is easy to handle. It is nevertheless subject to the variabilities inherent in a product derived from animal hides and processed to recover fibrous collagen.

It is accordingly an object of the present invention to provide an absorbable hemostatic material characterized by excellent physical and hemostatic properties.

It is a further object of the present invention to provide a wholly synthetic absorbable hemostatic material having a controlled absorption time.

It is a yet further object of the present invention to provide a synthetic absorbable hemostatic material in the form of a fabric having excellent handling and hemostatic properties.

These and other objects of the present invention will be apparent from the ensuing description and claims.

SUMMARY

An absorbable hemostatic fabric material is prepared from synthetic absorbable fibers comprising a homopolymer or copolymer of lactide and glycolide. The prepared fabric

J&J 1128

is exposed to gamma radiation in excess of 5 Mrads from cobalt-60 or an electron beam to increase the rate of absorption upon implantation. The original in vivo absorption time of the fabric may be from 50 to 90 days or more depending upon polymer composition, molecular weight, and processing conditions. The radiation dose is selected to decrease the in vivo absorption time to less than about 28 days, and most preferably to from 14 to 28 days.

The fabric may be woven or knitted, or formed as a nonwoven felt or fabric. A typical fabric is a knit tricot velour prepared from 3.3 Tex (30 denier) yarn on a 32 gauge tricot machine and having a density of 16 mg/cm². A fabric composed of polyglactin 910, a 90/10 copolymer of glycolide/lactide and exposed to 32.5 Mrad cobalt-60 radiation has an absorption time reduced from about 70 days to about 21 to 28 days.

The absorbable hemostatic fabrics of the present invention are characterized by excellent hemostatic properties and good handling characteristics. The suppleness of the knitted materials allows the hemostat to be formed around an arterial anastomosis where many other hemostatic materials have proven difficult to handle. The fabrics of the present invention may, if desired, be impregnated with thrombin, thromboplastin or other coagulating composition to enhance the hemostatic effect of the material.

DESCRIPTION OF DRAWINGS

Figure 1 is a plot illustrating effect of radiation exposure on absorption time of polyglactin 910 fibers.

Figure 2 is a plot illustrating effect of radiation exposure on the inherent viscosity of polyglactin 910 fibers.

0109197

## DETAILED DESCRIPTION OF INVENTION

The synthetic absorbable hemostats of the present invention comprise fibers or filaments of homopolymers and copolymers of lactide and glycolide. Such polymers have found wide use in the preparation of surgical sutures, and the preparation of the polymers and extrusion of filaments therefrom is well-known in the art. See, for example, U.S. Patents Nos. 2,668,162, Lowe; 2,676,945, Higgins; and 3,297,033, Schmitt.

The rate of absorption of the lactide and glycolide polymers depends primarily upon the composition of the polymer and the molecular weight. Absorption takes place as a result of hydrolysis which causes the polymer to revert to lactic acid and/or glycolic acid. In general, polylactide and copolymers comprising a major amount of lactide with a minor amount of glycolide have longer absorption times than polyglycolide, and high molecular weight polymers have longer absorption times than low molecular weight polymers.

One polymer composition which has been widely used as a surgical suture is polyglactin 910, a copolymer of 90% glycolide and 10% lactide. This composition, when extruded and drawn to form continuous filaments, is characterized by high tensile and knot strength, good handling characteristics, and an _in vivo_ absorption time of about 70 days.

The high tensile and knot strength of polyglactin 910 filaments are useful properties when preparing knitted or woven fabrics since yarns of such filaments are less prone to breakage than would be experienced with a lower strength material. In preparing the fabrics of the

J&J 1128

0109197

present invention, for example, we have found that polyglactin 910 yarns are readily processed on a tricot knitting machine to obtain fabrics having good hand and uniform knit construction.

Polyglactin 910 filaments absorb completely in about 70 days when implanted in humans. While such a rate of absorption is desirable for a surgical suture, it is significantly longer than the 14 to 28 days generally desired for a hemostatic product. Since absorption is known to occur through the mechanism of hydrolysis, the time for absorption to occur after implantation can be reduced by permitting partial hydrolysis to occur before implantation. This method has been suggested in connection with surgical dressings composed of polyglycolic acid filaments according to U.S. Patent No. 3,875,937. The hydrolysis reaction, however, is a function of time, temperature, humidity and other environmental conditions, and preconditioning hydrolytically unstable materials to reliably obtain a controlled degree of hydrolysis is difficult to achieve.

We have discovered that the time for absorption of surgical materials comprising homopolymers and copolymers of lactide and glycolide, including polyglactin 910, can be rapidly and consistently reduced in a controlled manner by exposing the material to a predetermined amount of radiation. Hemostatic fabrics constructed of suture quality filaments having, for example, 70-day absorption times are found to be completely absorbed in 21 days or less after exposure to 40 Mrads radiation from cobalt-60 or an electron beam.

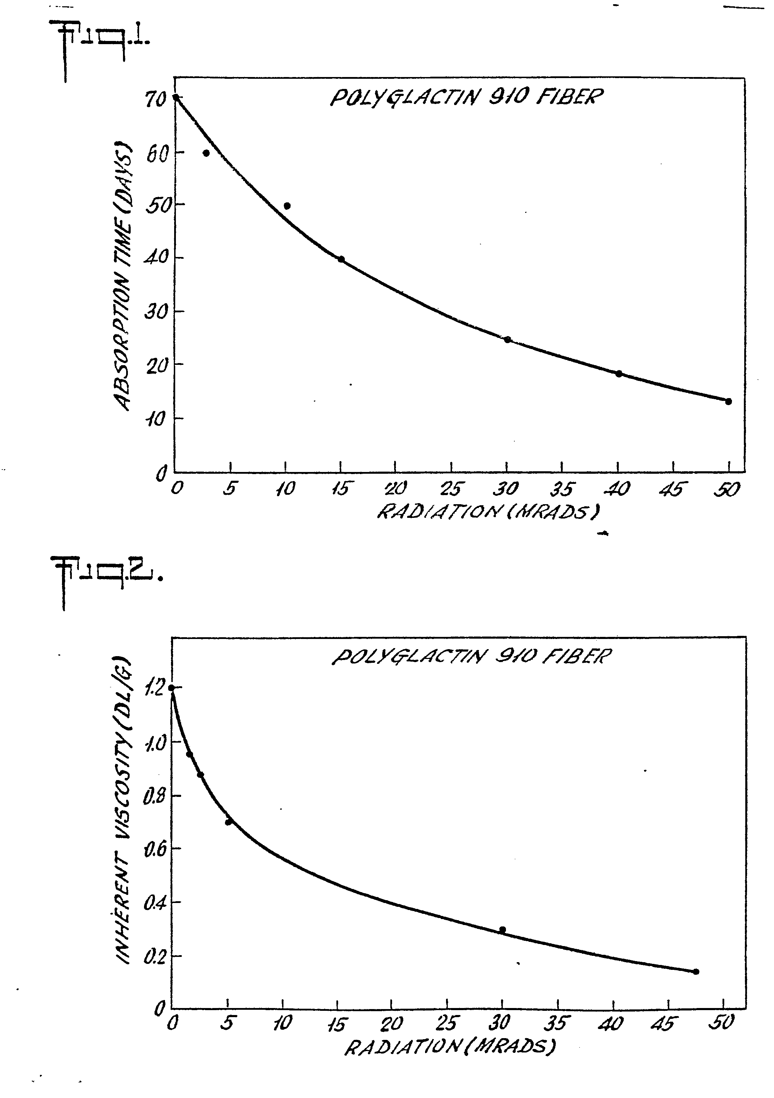

The amount of radiation required to obtain a desired absorption time is readily determined experimentally for any given lactide/glycolide polymer composition. In the

J&J 1128

0109197

case of polyglactin 910, the absorption time associated with increasing doses of cobalt-60 radiation is illustrated in FIGURE 1 and by the following data:

| Radiation dose, Mrad | : | 0 | 2.5 | 10 | 15 | 30 | 40 | 50 |
|---|---|---|---|---|---|---|---|---|
| Avg. absorption time, days: | | 70 | 60 | 50 | 40 | 25 | 19 | 14 |

A radiation dose of 2.5 Mrad has been established as a sterilizing exposure for surgical fibers and sutures. In a preferred method of the present invention, the hemostatic fabrics are prepared from suture-grade fibers on conventional textile knitting or weaving machines, or on nonwoven fabric machines, under nonsterile conditions. The finished fabrics are subjected to a dose of radiation of from about 5 to 45 Mrads selected to provide the desired absorption time. The irradiated fabrics are thereupon packaged in a hermetically sealed, moisture-impervious material such as aluminum foil, and sterilized in the package by exposure to an additional 2.5 Mrads of radiation. The final absorption rate of the fabric is that resulting from the sum of the initial dose of radiation plus the 2.5 Mrad sterilizing dose.

In preparing absorbable hemostats according to the present invention, care is taken to assure that preimplantation hydrolysis is kept to a minimum to avoid variations in such hydrolysis from causing variations in the final rate of absorption of the finished product. Yarns and fabrics are, for example, stored in a moisture-free environment from the time of extruding the filaments until packaging of the final product. Textile processing is preferably conducted in an environment with controlled humidity, and the times involved are sufficiently short and constant to avoid significant variation in the properties of the final product.

Polyglactin 910 sutures having an in vivo absorption time of about 70 days have an inherent viscosity of about 1.20 corresponding to a weight average molecular weight of about 30,000.  Exposure of this material to cobalt-60 or electron beam radiation results in a reduction of the inherent viscosity as a function of the total radiation dose as illustrated in FIGURE 2.  For use as an absorbable hemostat, the radiation dose is preferably selected to reduce the inherent viscosity to less than about 0.7, and most preferably to from about 0.15 to 0.30 which corresponds to an in vivo absorption time of approximately 14 to 28 days.  Inherent viscosity of the polymer is determined according to conventional analytical procedures using a capillary viscometer and a 0.1% (w/v) solution of polymer in dry hexafluoroisopropanol solvent, at 25°C.

The following data were obtained and are plotted in Figure 2:

| Radiation, Mrads | I.V., dl/g |
| --- | --- |
| 0 | 1.20 |
| 1.6 | 0.95 |
| 2.5 | 0.87 |
| 5.0 | 0.70 |
| 30.0 | 0.31 |
| 47.5 | 0.15 |

Polyglactin 910 fibers exposed to 47.5 Mrads radiation were additionally characterized by nuclear magnetic resonance (NMR) spectroscopy, and the number average molecular weight of such material calculated to be approximately 3000 daltons.

J&J 1128

The following examples illustrate the preparation of synthetic absorbable hemostats of polyglactin 910 in accordance with the present invention. The examples are presented for purposes of illustration only and are not to be construed as limiting of the invention.

EXAMPLE I

Continuous filament, untextured 3.3 Tex (30 denier) 8 filament yarn, of polyglactin 910 was converted into a closed, unbroken loop velour knit on a 32 gauge tricot machine. The front bar was set to knit 1-0/10-11 and the back bar was set to knit 1-2/1-0 with a quality of 16 cm (6-1/4 inches). Resulting fabric density was 16 mg/cm$^2$. The knit fabric was scoured with ethyl acetate to remove knitting lubricants, and cut into 7.6 x 12.7 cm ( 3 x 5 inch) pads. The pads were irradiated with 30 Mrads in a cobalt-60 radiation chamber and packaged in a dry environment in aluminum foil coated with a heat-sealable polymer film. The sealed packages were sterilized by exposure to 2.5 Mrads cobalt-60 radiation.

The knit fabric was evaluated as an absorbable hemostat in a splenic bleeding model animal test as follows: Mongrel dogs were anesthetized and prepared for surgery under aseptic conditions. The abdomen was shaved and a midline abdonimal incision made to gain access to the spleen. The spleen was mobilized into the surgical area and the upper and lower poles sequentially excised. Hemorrhage was controlled by finger compression until the hemostatic fabric could be applied. The hemostat was fixed with sutures and bleeding was observed to cease in about 4.5 minutes. Absorption time of this fabric as determined by subcutaneous implants in another dog was 14 to 21 days.

iJ 1128

## EXAMPLE II

A knit tricot velour fabric was prepared according to the method of Example I except the front and back bars of the machine were set at 1-0/3-4 and 1-2/1-0 respectively. The resulting fabric which was less dense than that of Example I had a density of 4 mg/cm$^2$. In the splenic bleeding model test, bleeding was observed to cease in about 6.5 minutes.

## EXAMPLE III

The knit fabric of Example I was irradiated with 15 Mrads of cobalt-60 radiation. In the splenic bleeding model test, bleeding was observed to cease in about 4.5 minutes. Subcutaneously implanted fabric samples were absorbed in 35-40 days.

## EXAMPLE IV

The knit fabric of Example I was irradiated with 30 Mrads of cobalt-60 radiation. Subcutaneous absorption occurred in 21 to 28 days.

## EXAMPLE V

A mongrel dog was prepared for surgery according to Example I and the abdominal aorta opened approximately 180 degrees. The incision was repaired loosely leaving major gaps along the suture line so there would be profuse bleeding. The knit fabric of Example I was applied by wrapping the vessel for a short time and the bleeding was completely controlled.

The knit hemostatic material of the preceding examples is particularly preferred over loose fiber and foam-type

J&J 1128

0109197

hemostatic materials because of its excellent handling characteristics. This is particularly significant where the hemostat is required to conform to an irregular or tubular surface as in the aortic bleeding test of Example V. The hemostatic products of the present invention are particularly preferred over collagen hemostats because of the uniformity made possible by the use of synthetic polymeric materials, and the ability to control absorption times. Particularly preferred are the tricot knit fabrics having a knit quality of 12.7 to 25.4 cm (5 to 10 inches) and fabric density of 10 to 30 mg/cm$^2$.

The hemostatic materials of the present invention may be impregnated with coagulant accelerators such as thrombin or thromboplastin, a mixture of tissue factors, phospholipids, proteins and lipids obtained as an extract from mammalian tissue, especially brains. Thromboplastin converts prothrombin to thrombin which causes blood to form a clot. The extract is widely used in hospital laboratories to perform the standard prothrombin time assay. Most mammalian tissues as well as red cells, platelets, soy beans, egg, yeast, fungi, and purified phosphatidyl amines have been reported to have thromboplastic activity.

Thrombin based coagulant solutions useful in the practice of the present invention are prepared by reconstituting commercially available lyophilized thrombin to a concentration of from 50 to 600 NIH units per ml with saline, water, or a pH 8.0-8.5 buffer solution, the specific concentration for any given hemostatic paid being selected to provide the desired concentration of thrombin in the final product. An NIH (U.S.) unit is defined as the amount of thrombin required to clot one milliliter of standardized fibrinogen solution in 15 seconds.

I&J 1128

0109197

Hemostatic materials impregnated with coagulant accelerators are obtained by saturating the material with a solution of the coagulant, freezing the saturated material, and subsequently lyophilizing the frozen material to obtain a dry product comprising a uniform distribution of the coagulant throughout the original material. The concentration of thrombin or other coagulating agent in the hemostatic material may be varied over a wide range since even a small amount of agent will be effective to provide some beneficial results. In general, however, at least 5 NIH thrombin units per $cm^2$ of hemostatic pad area, and preferably from 30-60 units, are recommended. While higher concentrations up to 100 units or more may provide marginally improved results, the extent of the improvement is generally not significant in terms of practical application and cost.

Because the hemostatic materials of the present invention have a chemical composition which is sensitive to moisture, care must be taken during impregnation with the coagulant accelerator to avoid undue hydrolytic degradation of the underlying material. This can be achieved by expeditious processing which minimizes the time during which the material is exposed to wet or humid conditions.

While the preceding examples have been directed to tricot knit fabrics of polyglactin 910 as a preferred embodiment of the present invention, woven, nonwoven, flocked and other knit types of fabric may also be utilized. In addition, other polymers of lactide and glycolide, and other sources of radiation may be utilized with satisfactory results as hereinbefore described. These and other variations in the practice of the present invention are within the ability of those skilled in the art and are included within the scope of the present invention.

J&J 1128

0109197

## CLAIMS

1. An absorbable hemostatic material comprising a sterile fabric constructed of synthetic absorbable filaments comprising homopolymers or copolymers of lactide and glycolide, said filaments having an in vivo absorption time of about 70 days, said fabric being exposed to radiation energy in excess of 5 Mrads whereby the in vivo absorption time of said fabric is reduced to less than about 28 days.

2. A hemostatic material according to claim 1, wherein said fabric is a closed-loop, knitted, tricot velour.

3. A hemostatic material according to claim 1 or claim 2, which after radiation exposure has an in vivo absorption time of from 14 to 28 days.

4. A hemostatic material according to any one of claims 1-3, constructed of absorbable filaments comprising a copolymer of about 90 parts glycolide and 10 parts lactide.

5. A hemostatic material according to claim 4, wherein said copolymer has an inherent viscosity of at least 1.2 before radiation exposure and less than 0.7 after radiation.

6. A hemostatic material according to any one of claims 1 to 5, further comprising a coagulating agent distributed uniformly throughout the body of said fabric.

7. A hemostatic material according to claim 6, wherein said coagulating agent is a thrombin or thromboplastin.

8. A hemostatic material according to claim 7, wherein the concentration of said coagulating agent in said hemostatic material is more than 5 NIH thrombin units per square centimeter of said fabric.

9. A hemostatic material according to any one of claims 1 to 8, packaged in a moisture-free environment in a hermetically sealed, moisture impervious material.

10. A method for reducing the in vivo absorption time of a hemostatic material comprising a fabric constructed of synthetic absorbable filaments comprising homopolymers or copolymers of lactide and glycolide, which method comprises exposing said fabric to radiation in excess of 5 Mrads.

1/1

0109197

Fig.1.

POLYGLACTIN 910 FIBER

ABSORPTION TIME (DAYS) vs RADIATION (MRADS)

Fig.2.

POLYGLACTIN 910 FIBER

INHERENT VISCOSITY (DL/G) vs RADIATION (MRADS)